# EUROPEAN PATENT SPECIFICATION

(11) **EP 3 956 051 B1**
(45) Date of publication and mention of the grant of the patent: **01.03.2023**
(21) Application number: 20725925.0
(22) Date of filing: 17.04.2020
(51) Int. Cl.: B01J 4/00, B01J 4/02, B01J 8/08, B01J 10/00, F04B 13/02, C07C 273/04

(54) **SYSTEM AND METHOD FOR FEEDING A UREA SYNTHESIS REACTOR**
SYSTEM UND VERFAHREN ZUR SPEISUNG EINES HARNSTOFFSYNTHESEREAKTORS
SYSTÈME ET PROCÉDÉ D'ALIMENTATION D'UN RÉACTEUR DE SYNTHÈSE D'URÉE

(30) Priority: 17.04.2019 IT 201900006002
(43) Date of publication of application: 23.02.2022
(73) Proprietor: Saipem S.p.A., 20138 Milano (IT)
(72) Inventor: VIGANO', Luca Edoardo, 20097 San Donato Milanese (MI) (IT); CARLESSI, Lino, 20097 San Donato Milanese (MI) (IT)
(74) Representative: Cernuzzi, Daniele
(86) International application number: PCT/IB2020/053654
(87) International publication number: WO 2020/212926

(56) References cited:
- WO-A1-2016/090198
- WO-A1-2017/171546
- Jozef H. Meessen: "Urea" In: "Ullmann's Encyclopedia of Industrial Chemistry", 15 October 2010 (2010-10-15), Wiley-VCH Verlag, Weinheim, XP055017973, ISBN: 978-3-52-730673-2 DOI: 10.1002/14356007.a27_333.pub2, page 24/40; figure 29

## Description

### TECHNICAL FIELD

The present invention relates to a system and a method for feeding a urea synthesis reactor.

In particular, the invention relates to a urea synthesis reactor provided with a feeding system for feeding the reactor with ammonia, carbon dioxide and non-converted reactants recovered downstream of the reactor; and a method for feeding a urea synthesis reactor with ammonia, carbon dioxide and non-converted reactants recovered downstream of the reactor.

### STATE OF THE PRIOR ART

As is known, urea is produced on an industrial scale through processes based on the reaction, under conditions of high temperature and high pressure, between carbon dioxide and ammonia forming ammonium carbamate (intermediate), and on the subsequent decomposition reaction of ammonium carbamate forming urea and water.

The urea synthesis reaction is carried out in a reactor from which an aqueous urea solution is obtained, which is then progressively concentrated, with recovery and recycling of the non-converted reactants, and solidified in a finishing section (for example, a granulator or a prilling tower).

The urea solution leaving the reactor contains non-converted reactants, which are recovered in different ways, depending also on the plant scheme and on the process adopted, for being then fed back to the reactor.

A typical urea synthesis reactor of a urea plant is therefore supplied with an essentially gas flow of carbon dioxide and an essentially liquid flow of ammonia and ammonium carbamate in solution.

The carbamate is normally recovered in aqueous solution at a pressure which is not sufficient for being fed to the bottom of the reactor, which is typically found at higher pressures.

For the recycling of the carbamate to the reactor, in particular, as is known, an ejector (carbamate ejector) is used.

Thanks to the geometry of the ejector, the carbamate to be recycled to the reactor is sucked up and recycled to the reactor exploiting the loss of kinetic energy of the ammonia at high pressure which acts as driving fluid and is also fed to the reactor.

The ejector is an apparatus which, although extremely simple and reliable being free of moving mechanical parts, is characterized by a low efficiency: in order to be able to recycle the carbamate to the reactor, even where the pressure drop is relatively small, the driving fluid (ammonia) pressure demand at the ejector inlet necessary for the entrainment of the carbamate is very high, with a pressure drop between inlet and outlet being also significant.

Especially for large plants, the use of an ejector can therefore be highly limiting in terms of efficiency and energy consumption, as well as availability on the market.

WO2016090198A1 discloses a urea plant in which the urea synthesis reactor is provided with a feeding system comprising a turbocharger. In some embodiments, the turbocharger receives a urea synthesis stream at high pressure and an ammonium carbamate stream at low pressure, and is used to pressurize the ammonium carbamate stream, in order to reduce wear to the high pressure carbamate pump, thus reducing the downtime and capital costs associated with replacing and/or repairing the high pressure carbamate pump and reducing the energy costs of the urea synthesis system.

### OBJECT OF THE INVENTION

It is an object of the present invention to overcome the drawbacks of the prior art highlighted above; in particular, it is an object of the invention to provide a system and a method for feeding a urea synthesis reactor, and consequently a plant and a method for producing urea, capable of having improved efficiency with respect to the prior art, allowing in particular a reduction in energy consumption.

The present invention therefore relates to a urea synthesis reactor provided with a feeding system for feeding the reactor with ammonia, carbon dioxide and non-converted reactants recovered downstream of the reactor, and a method for feeding a urea synthesis reactor with ammonia, carbon dioxide and non-converted reactants recovered downstream of the reactor, as defined in essential terms in the appended Claims 1 and 7, respectively.

The invention also relates to a urea plant as defined by Claim 6; as well as a process for the synthesis of urea by direct biphasic reaction of ammonia and carbon dioxide at high temperature and high pressure, as defined in Claim 13.

Preferred additional features of the invention are indicated in the dependent claims.

The invention provides a plant and a method for producing urea which are particularly efficient in terms of energy consumption, in particular in the feeding of the urea synthesis reactor.

Instead of an ejector or other poorly efficient apparatus, in accordance with the invention, a turbocharger is used for the recycling of non-converted reactants (carbamate) to the reactor: as in an ejector, also in the turbocharger, the ammonia is exploited as driving fluid, but in this case the ammonia transfers part of its kinetic energy to the carbamate, supplying the energy necessary for pumping the carbamate into the reactor, acting on a first impeller connected to a second impeller which increases the pressure of the carbamate.

The use of a turbocharger allows to reduce the pressure required for the ammonia and the relative pressure drop compared to plant configurations with an ejector, reducing energy consumption and thus improving efficiency.

The turbocharger does not present any sealing problems, since the shaft that connects the two impellers of the turbocharger is not externally connected to any engine, and does not require a forced separation of the two flows in transit, which can partly mix without substantial reductions in efficiency.

In addition to reducing energy consumption, the solution of the invention also allows to keep the flows of ammonia and carbamate fed into the reactor separate.

In this way, unlike with an ejector, the ammonia and carbamate streams downstream of the turbocharger remain separate and can thus be fed to the reactor in optimal positions.

In particular, this makes it possible to feed ammonia not only into the bottom of the reactor but also along the vertical extension of the same, in an optimal position to facilitate the conversion.

Carbamate can also be fed at an optimal reactor height. Furthermore, while in known plant configurations the carbamate is sent in full to the carbamate condensers, it is possible to send part of the carbamate directly from the high-pressure pumps to the reactor, thus allowing for greater operational flexibility.

### BRIEF DESCRIPTION OF THE DRAWINGS

Further characteristics and advantages of the present invention will become clear from the description of the following non-limiting embodiment examples, with reference to the figures of the attached drawings, wherein:
- Figure 1 is a schematic view of a urea production plant (urea plant) according to the invention;
- Figure 2 is a schematic view in greater detail of a portion of the urea plant of Figure 1;
- Figure 3 is a schematic view of an apparatus (in particular, a turbocharger) used in the urea plant of Figures 1 and 2;
- Figure 4 shows variants of the urea plant of the invention.

### PREFERRED EMBODIMENT OF THE INVENTION

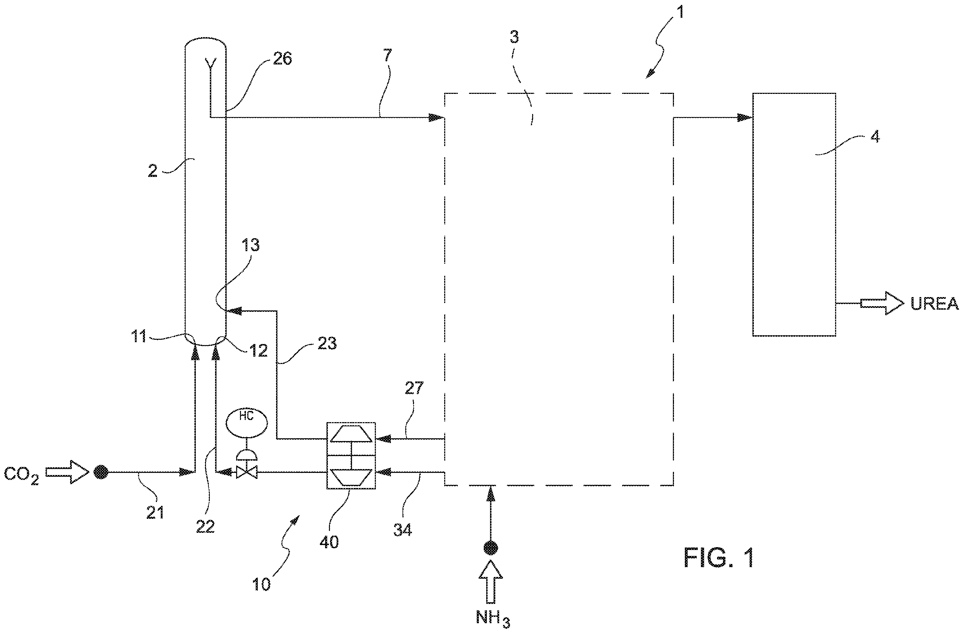

Figure 1 shows an extremely schematic view of a urea plant 1 (i.e. a plant for the production of urea), operating for carrying out a process for the synthesis of urea by direct biphasic reaction of ammonia and carbon dioxide at high temperature and high pressure.

The general configuration of the urea plant 1 can be of various types, as well as the urea production process carried out in the plant.

For example, but not necessarily, the urea plant 1 is of the type operating essentially according to the technology known as "Snamprogetti^{™} Urea Technology", to which the changes described below are applied for the implementation of the present invention. However, it is understood that the invention also applies to other urea production plants/processes.

In general, the urea plant 1 comprises: a urea synthesis reactor 2 wherein a urea synthesis reaction from ammonia and carbon dioxide takes place; treatment sections 3, shown as a whole with dotted lines in Figure 1, which can have various configurations (comprising for example: a high pressure recovery section, a medium pressure recovery section, a low pressure recovery section, a vacuum concentration section connected to a process condensate treatment section), wherein a urea solution produced in the reactor 2 is progressively concentrated with removal therefrom of unreacted ammonia and carbon dioxide and water and recirculation of the recovered components; a finishing/solidification section 4 comprising, for example, a granulator or a prilling tower.

A urea line 7 brings a urea solution produced in the reactor 2 to the treatment sections 3, where the urea is progressively concentrated and separated from the non-converted reactants, before being sent to the finishing/solidification section 4.

Here and in the following, not all the components of the various sections and of the circuits which connect them are indicated and described, but only those useful for the understanding of the present invention.

The reactor 2 is fed with the reactants required for the urea synthesis reaction, i.e. CO2 and NH3, as well as with non-converted reactants recovered from the treatment sections 3 and recirculated to the reactor 2, via a feeding system 10.

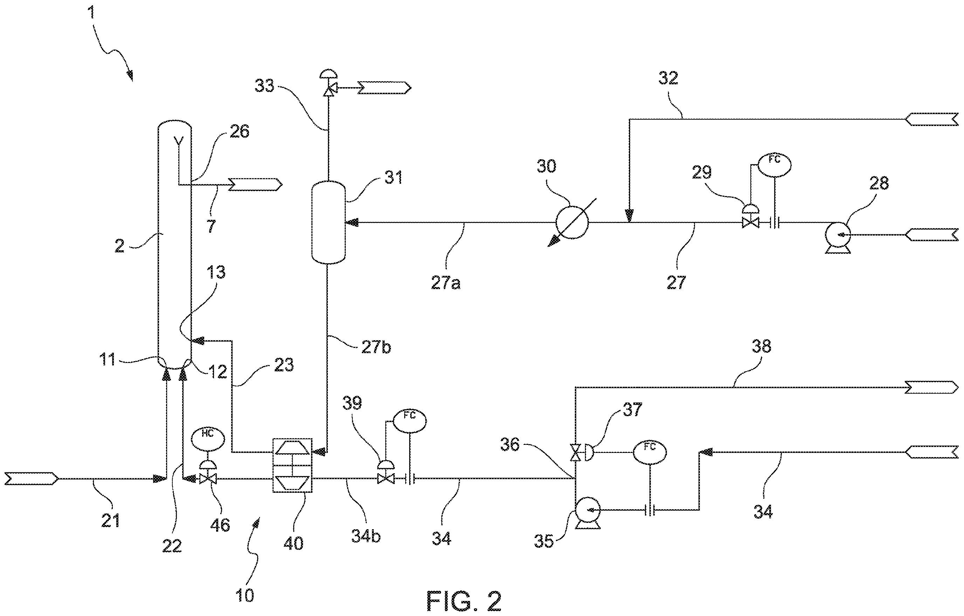

In greater detail and with reference also to Figure 2, the reactor 2 has a first inlet 11, a second inlet 12 and a third inlet 13, connected to respective feeding lines 21, 22, 23 of the feeding system 10 and precisely: a CO2 feeding line 21, an ammonia feeding line 22, and a carbamate feeding line 23 (here and in the following, ammonium carbamate).

The inlets 11, 12, 13 are distinct and separate from one another and positioned in different positions on the reactor 2, preferably in a lower portion of the reactor 2 if the latter, as in the example shown, has a substantially vertical development (i.e. it extends along a vertical longitudinal axis in use).

In particular, the inlet 11 connected to the CO2 feeding line 21 and the inlet 12 connected to the ammonia feeding line 22 are, for example, positioned at a lower end of the reactor 2, through a bottom wall, for example shaped like a cap, of the reactor 2.

In the non-limiting example illustrated, the inlet 13 connected to the carbamate feeding line 23 is positioned higher in the reactor 2 (at a higher level, i.e. at a greater height along the longitudinal axis of the reactor 2) with respect to the inlet 12 connected to the ammonia feeding line 22, in particular through a side wall, for example of substantially cylindrical shape, of the reactor 2.

However, it is understood that the inlets 11, 12, 13 can be positioned differently than as shown herein by way of example only. In particular, the inlet 13 connected to the carbamate feeding line 23 does not necessarily have to be positioned higher than the inlet 12 connected to the ammonia feeding line 22, and can be positioned lower or even substantially at the same height, for example this too through the bottom wall of the reactor 2, of the inlet 12.

Furthermore, the reactor has an outlet 26, positioned at an upper end of the reactor 2 and connected to the urea line 7, from which an aqueous urea solution containing non-converted reactants is withdrawn from the reactor 2.

The urea line 7 connects the outlet 26 to the treatment sections 3 (known and not illustrated), where the urea solution produced in the reactor 2 is progressively concentrated and the non-converted reactants, in particular carbamate (ammonium carbamate), are recovered.

The methods for recovering carbamate (and in general non-converted reactants) can be various, also depending on the urea process carried out in the plant 1, and require variously configured and connected components.

The carbamate recovered in the treatment sections 3 is then sent to the reactor 2 through the carbamate feeding line 23.

In the purely exemplary embodiment illustrated in particular in Figure 2, the carbamate (understood, here and in the following, as a solution containing carbamate) is withdrawn from the treatment sections 3 via a carbamate line 27, equipped with a high pressure pump 28 and a flow rate control valve 29 (associated with a flow controller FC), and sent into a carbamate condenser 30 (defined in particular by a heat exchanger) and into a carbamate separator 31, arranged in series along the carbamate line 27 and connected by a section 27a of the carbamate line 27.

Optionally, on the carbamate line 27 a vapor line 32 is inserted, upstream of the carbamate condenser 30, which adds a gas flow containing process vapors, also recovered from the treatment sections 3, to the carbamate solution circulating in the carbamate line 27.

The carbamate separator 31 is configured for carrying out the separation of a liquid phase containing carbamate from a gas phase, which is removed from a separator head outlet through a gas discharge line 33.

The liquid phase exits from a bottom outlet of the carbamate separator 31 and constitutes an essentially liquid stream containing carbamate (carbamate stream) which is sent to a further section 27b of the carbamate line 27 to be fed to the reactor 2.

Indicatively, the carbamate stream leaving the carbamate separator 31 and circulating in the section 27b has a pressure comprised between about 140 bar (g) and about 150 bar (g).

An ammonia line 34, equipped with one or more high pressure pumps 35, brings ammonia, precisely an essentially liquid stream containing ammonia (ammonia stream), to the ammonia feeding line 22.

Advantageously, the ammonia line 34 is provided with a ramification 36 controlled by a flow rate control valve 37 (associated with a flow controller FC) and connected via an auxiliary line 38 to the treatment sections 3, so as to split the ammonia between a first stream, which feeds the reactor 2, and a second stream, which is sent to the treatment sections 3.

Indicatively, the ammonia circulating in the ammonia line 34 downstream of the high pressure pump 35 has a pressure comprised between about 190 bar (g) and about 210 bar (g).

Furthermore, the line 34 is optionally provided with a flow rate control valve 39 (associated with a flow controller FC) positioned downstream of the pump 35 to possibly reduce the ammonia pressure in a final section 34b of the line 34.

According to the invention, the feeding system 10 comprises a turbocharger 40, which is used for feeding ammonia and carbamate to the reactor 2.

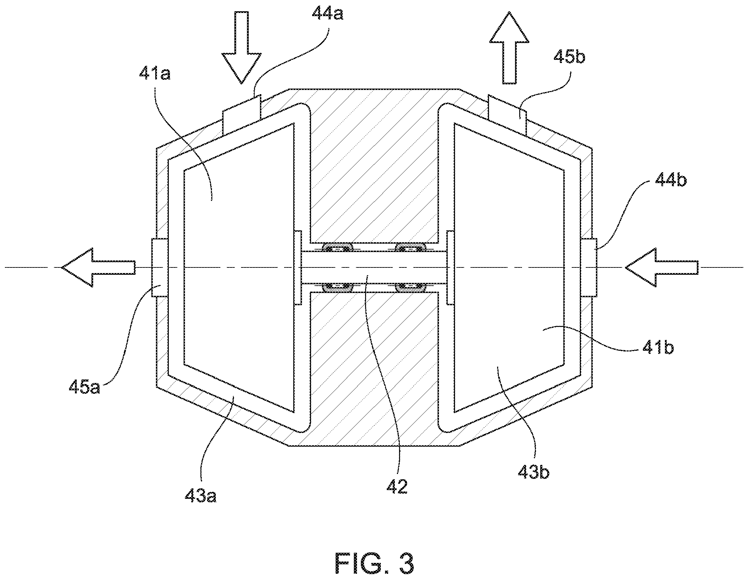

The turbocharger 40 is an apparatus (precisely, a turbomachine), schematically represented in Figure 3, essentially comprised of two impellers 41a, 41b integrally connected to each other by a rotating shaft 42. The impellers 41a, 41b are rotatably housed in respective separate housings 43a, 43b, equipped with respective inlets 44a, 44b, and respective outlets 45a, 45b.

In accordance with the invention, the inlets 44a, 44b of the turbocharger 40 are connected to the ammonia line 34 (precisely, to its final section 34b) and to the carbamate line 27 (precisely, to its section 27b), respectively; the outputs 45a, 45b are connected to the ammonia feeding line 22 and to the carbamate feeding line 23, respectively. Therefore, the turbocharger 40 has an ammonia-side impeller 41a and a carbamate-side impeller 41b.

The ammonia-side impeller 41a is rotationally driven by the passage of the ammonia stream fed to the turbocharger 40 through the ammonia line 34 and transmits the rotation, via the shaft 42, to the carbamate-side impeller 41b, which acts on the carbamate stream supplied through the carbamate line 27 and increases the pressure thereof.

The ammonia acts as driving fluid in the turbocharger 40, transferring part of its kinetic energy to the carbamate and thus supplying the energy necessary for pumping the carbamate into the reactor 2 at the required pressure.

Indicatively, but not necessarily, the turbocharger 40 operates with an ammonia stream having an inlet pressure comprised between about 160 bar (g) and about 190 bar (g), and an outlet pressure comprised between about 150 bar (g) and about 160 bar (g); and a carbamate stream having an inlet pressure comprised between about 140 bar (g) and about 150 bar (g), and an outlet pressure comprised between about 150 bar (g) and about 160 bar (g).

The ammonia stream, after passing through the turbocharger 40, where it transfers part of its kinetic energy, is fed into the reactor 2 through the ammonia feeding line 22, optionally equipped with a control valve 46.

The carbamate stream, after passing through the turbocharger 40 and being brought to the required pressure, is fed into the reactor 2, separately from the ammonia stream, through the carbamate feeding line 23, in a different position in the reactor 2, for example (but not necessarily) at a higher height with respect to ammonia. As already highlighted, the carbamate stream can be fed into the reactor 2 at a higher, lower or even substantially equal height with respect to ammonia.

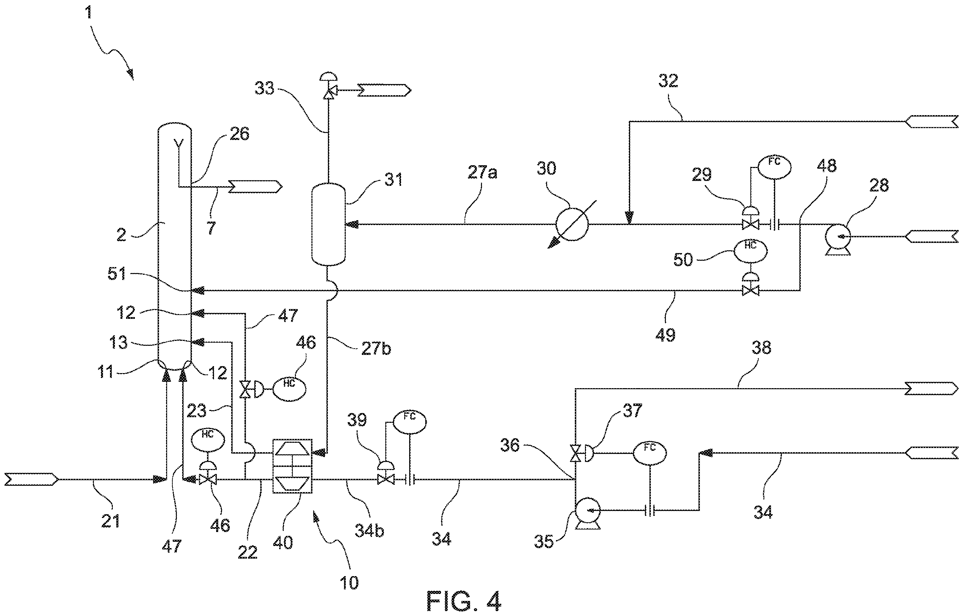

In the variant shown in Figure 4, the ammonia feeding line 22 divides, downstream of the turbocharger 40, in two (or more) ammonia feeding branches 47, equipped with respective control valves 46 and connected to respective inlets 12 of the reactor 2.

In this way, the ammonia can be fed into the reactor 2 in different positions, not only on the bottom of the reactor 2, but also at different heights along the reactor 2.

According to a further variant, also illustrated in Figure 4, the carbamate line 27 has, downstream of the high pressure pump 28, a branch 48 connected to an auxiliary carbamate line 49. The auxiliary carbamate line 49, equipped with a control valve 50, is connected directly to the reactor 2, in particular to a supplementary inlet 51 of the reactor 2.

In this way, a carbamate portion is sent directly to the reactor 2 by the high pressure pump 28.

Clearly, the variants just described can also be used separately from each other.

Finally, it is understood that further modifications and variations can be made to the plant and method described and illustrated herein, that do not fall outside the scope of the attached claims.

## Claims

1. A urea synthesis reactor (2) provided with a feeding system (10) for feeding the reactor (2) with ammonia, carbon dioxide and non-converted reactants recovered downstream of the reactor (2), the feeding system (10) comprising a first inlet (11) connected to a CO2 feeding line (21) and a second inlet (12) connected to an ammonia feeding line (22); the reactor (2) being **characterized in that** the feeding system (10) comprises a turbocharger (40), which is used to supply ammonia and ammonium carbamate to the reactor (2) and has two impellers (41a, 41b) rotatably housed in respective housings (43a, 43b) and integrally connected to each other by a rotating shaft (42); the housings (43a, 43b) having respective inlets (44a, 44b), connected to an ammonia line (34) and a carbamate line (27), respectively, to bring to the turbocharger (40) and to the respective impellers (41a, 41b) a first and a second essentially liquid streams containing ammonia and carbamate, respectively; and respective outlets (45a, 45b) connected to the reactor (2); wherein the impellers (41a, 41b) of the turbocharger (40) define an ammonia-side impeller (41a) and a carbamate-side impeller (41b), respectively; the ammonia-side impeller (41a) being driven by the passage of the first stream, containing ammonia, supplied to the turbocharger (40) through the ammonia line (34); the carbamate-side impeller (41b) being rotationally driven, via the shaft (42), by the ammonia-side impeller (41a) and acting on the second stream, containing carbamate, supplied through the carbamate line (27) to increase the pressure thereof.

2. The reactor according to Claim 1, wherein the outlets (45a, 45b) of the turbocharger (40) are connected to said second inlet (12) of the reactor (2), via the ammonia feeding line (22), and to a third inlet (13) of the reactor (2), via the carbamate feeding line (23), respectively.

3. The reactor according to Claim 2, wherein said third inlet (13), connected to the carbamate feeding line (23), is positioned in a different position, for example a higher position, in the reactor (2) with respect to said second inlet (12), connected to the ammonia feeding line (22).

4. The reactor according to any one of the preceding claims, wherein the ammonia feeding line (22) divides, downstream of the turbocharger (40), in two or more ammonia feeding branches (47) connected to respective inlets (12) of the reactor (2), so as to feed ammonia to the reactor (2) in different positions.

5. The reactor according to any one of the preceding claims, wherein the carbamate line (27) has a ramification (48) connected to an auxiliary carbamate line (49) which is connected directly to the reactor (2) and does not pass through the turbocharger (40), in particular to a supplementary inlet (51) of the reactor (2).

6. A urea plant (1), **characterized in that** it comprises the reactor (2) and the associated feeding system according to any one of the preceding claims.

7. A method for feeding a urea synthesis reactor (2) with ammonia, carbon dioxide and non-converted reactants recovered downstream of the reactor (2), comprising the steps of feeding CO2 and ammonia to the reactor (2) via a first inlet (11) and a second inlet (12), respectively; the method being **characterized by** supplying a first essentially liquid stream comprising ammonia to a first impeller (41a) which is rotatably driven by said first stream acting as driving fluid; supplying a second essentially liquid stream comprising carbamate to a second impeller (41b), integrally connected to the first impeller by a rotating shaft (42), to increase the pressure of said second stream; supplying said first and second streams, after cooperating with said impellers (41a, 41b), to the reactor (2), whereby the impellers are of a turbocharger (40) as defined in claim 1.

8. The method according to Claim 7, wherein the impellers (41a, 41b) define an ammonia-side impeller (41a) and a carbamate-side impeller (41b), respectively; the ammonia-side impeller (41a) being driven by the passage of said first stream; the carbamate-side impeller (41b) being rotationally driven, via the shaft (42), by the ammonia-side impeller (41a) and acting on said second stream to increase the pressure thereof.

9. The method according to Claim 7 or 8, wherein said first stream and said second stream, after cooperating with said impellers (41a, 41b), are supplied to the reactor (2) separately from each other.

10. The method according to Claim 9, wherein the second stream, containing carbamate, is fed into the reactor (2) in a different position, for example a higher position, with respect to the first stream.

11. The method according to any one of Claims 7 to 10, comprising a step of dividing said first stream, after cooperating with said first impeller (41a), in two or more streams which are fed to the reactor (2) in different positions.

12. The method according to any one of Claims 7 to 11, comprising the step of supplying to the reactor (2) a further essentially liquid stream containing carbamate separately from said second stream and without passing through said second impeller (41b).

13. A urea synthesis process by direct biphasic reaction of ammonia and carbon dioxide at high temperature and high pressure, comprising a step of feeding to a urea synthesis reactor (2) ammonia, carbon dioxide and non-converted reactants recovered downstream of the reactor (2); **characterized in that** said step of feeding to the urea synthesis reactor (2) ammonia, carbon dioxide and non-converted reactants recovered downstream of the reactor (2) is carried out by the method according to any one of Claims 7 to 12.

## Patentansprüche

1. Harnstoffsynthesereaktor (2), der mit einem Speisesystem (10) zum Speisen des Reaktors (2) mit Ammoniak, Kohlendioxid und nicht umgesetzten Reaktanten, die stromabwärts des Reaktors (2) zurückgewonnen werden, versehen ist, wobei das Speisesystem (10) einen ersten Einlass (11), der mit einer CO₂-Speiseleitung (21) verbunden ist, und einen zweiten Einlass (12), der mit einer Ammoniak-Speiseleitung (22) verbunden ist, umfasst; wobei der Reaktor (2) **dadurch gekennzeichnet ist, dass** das Speisesystem (10) einen Lader (40) umfasst, der zum Zuführen von Ammoniak und Ammoniumcarbamat in den Reaktor (2) verwendet wird und zwei Laufräder (41a, 41b) aufweist, die drehbar in jeweiligen Gehäusen (43a, 43b) aufgenommen sind und durch eine Drehwelle (42) einstückig miteinander verbunden sind; wobei die Gehäuse (43a, 43b) jeweilige Einlässe (44a, 44b) aufweisen, die jeweils mit einer Ammoniakleitung (34) und einer Carbamatleitung (27) verbunden sind, um dem Lader (40) und den jeweiligen Laufrädern (41a, 41b) einen ersten und einen zweiten im Wesentlichen flüssigen Strom zuzuführen, die jeweils Ammoniak und Carbamat enthalten; und jeweilige Auslässe (45a, 45b) aufweisen, die mit dem Reaktor (2) verbunden sind; wobei die Laufräder (41a, 41b) des Laders (40) jeweils ein ammoniakseitiges Laufrad (41a) und ein carbamatseitiges Laufrad (41b) definieren; wobei das ammoniakseitige Laufrad (41a) durch den Durchtritt des ersten Stroms, der Ammoniak enthält, welcher dem Lader (40) durch die Ammoniakleitung (34) hindurch zugeführt wird, angetrieben wird; wobei das carbamatseitige Laufrad (41b) durch das ammoniakseitige Laufrad (41a), über die Welle (42), drehangetrieben wird und so auf den zweiten Strom, der Carbamat enthält, welcher durch die Carbamatleitung (27) hindurch zugeführt wird, wirkt, dass es dessen Druck erhöht.

2. Reaktor nach Anspruch 1, wobei die Auslässe (45a, 45b) des Laders (40) jeweils über die Ammoniak-Speiseleitung (22) mit dem zweiten Einlass (12) des Reaktors (2) und über die Carbamat-Speiseleitung (23) mit einem dritten Einlass (13) des Reaktors (2) verbunden sind.

3. Reaktor nach Anspruch 2, wobei der dritte Einlass (13), der mit der Carbamat-Speiseleitung (23) verbunden ist, in Bezug auf den zweiten Einlass (12), der mit der Ammoniak-Speiseleitung (22) verbunden ist, an einer anderen Position, beispielsweise einer höhergelegenen Position, in dem Reaktor (2) positioniert ist.

4. Reaktor nach einem beliebigen der vorhergehenden Ansprüche, wobei sich die Ammoniak-Speiseleitung (22), stromabwärts des Laders (40), in zwei oder mehr Ammoniak-Speiseabzweigungen (47) aufteilt, die mit jeweiligen Einlässen (12) des Reaktors (2) verbunden sind, um Ammoniak an unterschiedlichen Positionen in den Reaktor (2) zu speisen.

5. Reaktor nach einem beliebigen der vorhergehenden Ansprüche, wobei die Carbamatleitung (27) eine Gabelung (48) aufweist, die mit einer Carbamat-Nebenleitung (49) verbunden ist, welche direkt mit dem Reaktor (2), insbesondere mit einem zusätzlichen Einlass (51) des Reaktors (2), verbunden ist und nicht durch den Lader (40) hindurchgeht.

6. Harnstoffanlage (1), **dadurch gekennzeichnet, dass** sie den Reaktor (2) und das zugehörige Speisesystem nach einem beliebigen der vorhergehenden Ansprüche umfasst.

7. Verfahren zum Speisen eines Harnstoffsynthesereaktors (2) mit Ammoniak, Kohlendioxid und nicht umgesetzten Reaktanten, die stromabwärts des Reaktors (2) zurückgewonnen werden, umfassend die Schritte des Speisens von CO₂ und Ammoniak jeweils über einen ersten Einlass (11) und einen zweiten Einlass (12) in den Reaktor (2); wobei das Verfahren **gekennzeichnet ist durch** Zuführen eines ersten im Wesentlichen flüssigen Stroms, der Ammoniak umfasst, zu einem ersten Laufrad (41a), das dadurch drehangetrieben wird, dass der erste Strom als Antriebsfluid agiert; Zuführen eines zweiten im Wesentlichen flüssigen Stroms, der Carbamat umfasst, zu einem zweiten Laufrad (41b), das mit dem ersten Laufrad durch eine Drehwelle (42) einstückig verbunden ist, um den Druck des zweiten Stroms zu erhöhen; Zuführen des ersten und des zweiten Stroms, nach Zusammenwirken mit den Laufrädern (41a, 41b), zu dem Reaktor (2), wobei die Laufräder Teil eines Laders (40) wie in Anspruch 1 definiert sind.

8. Verfahren nach Anspruch 7, wobei die Laufräder (41a, 41b) jeweils ein ammoniakseitiges Laufrad (41a) und ein carbamatseitiges Laufrad (41b) definieren; wobei das ammoniakseitige Laufrad (41a) durch den Durchtritt des ersten Stroms angetrieben wird; wobei das carbamatseitige Laufrad (41b), über die Welle (42), durch das ammoniakseitige Laufrad (41a) drehangetrieben wird und auf den zweiten Strom wirkt, um dessen Druck zu erhöhen.

9. Verfahren nach Anspruch 7 oder 8, wobei der erste Strom und der zweite Strom, nach Zusammenwirken mit den Laufrädern (41a, 41b), dem Reaktor (2) getrennt voneinander zugeführt werden.

10. Verfahren nach Anspruch 9, wobei der zweite Strom, der Carbamat enthält, an einer in Bezug auf den ersten Strom anderen Position, beispielsweise einer höhergelegenen Position, in den Reaktor (2) gespeist wird.

11. Verfahren nach einem beliebigen der Ansprüche 7 bis 10, umfassend einen Schritt des Aufteilens des ersten Stroms, nach Zusammenwirken mit dem ersten Laufrad (41a), in zwei oder mehr Ströme, die an unterschiedlichen Positionen in den Reaktor (2) gespeist werden.

12. Verfahren nach einem beliebigen der Ansprüche 7 bis 11, umfassend den Schritt des Zuführens eines weiteren im Wesentlichen flüssigen Stroms, der Carbamat enthält, getrennt von dem zweiten Strom und ohne Hindurchtreten durch das zweite Laufrad (41b), zu dem Reaktor (2).

13. Prozess zur Harnstoffsynthese durch direkte biphasige Reaktion von Ammoniak und Kohlendioxid bei hoher Temperatur und hohem Druck, umfassend einen Schritt des Speisens, in einen Harnstoffsynthesereaktor (2), von Ammoniak, Kohlendioxid und nicht umgesetzten Reaktanten, die stromabwärts des Reaktors (2) zurückgewonnen werden; **dadurch gekennzeichnet, dass** der Schritt des Speisens, in den Harnstoffsynthesereaktor (2), von Ammoniak, Kohlendioxid und nicht umgesetzten Reaktanten, die stromabwärts des Reaktors (2) zurückgewonnen werden, durch das Verfahren nach einem beliebigen der Ansprüche 7 bis 12 durchgeführt wird.

## Revendications

1. Réacteur de synthèse d'urée (2) pourvu d'un système d'alimentation (10) pour alimenter le réacteur (2) en ammoniac, en dioxyde de carbone et en réactifs non convertis récupérés en aval du réacteur (2), le système d'alimentation (10) comprenant une première entrée (11) reliée à une conduite d'alimentation en CO2 (21) et une deuxième entrée (12) reliée à une conduite d'alimentation en ammoniac (22) ; le réacteur (2) étant **caractérisé en ce que** le système d'alimentation (10) comprend un turbocompresseur (40), qui est utilisé pour fournir de l'ammoniac et du carbamate d'ammonium au réacteur (2) et comporte deux turbines (41a, 41b) logées de manière à pouvoir tourner dans des logements respectifs (43a, 43b) et reliées solidairement l'une à l'autre par un arbre de rotation (42) ; les logements (43a, 43b) comportant des entrées (44a, 44b) respectives, reliées respectivement à une conduite d'ammoniac (34) et à une conduite de carbamate (27), pour amener au turbocompresseur (40) et aux turbines (41a, 41b) respectives des premier et deuxième flux sensiblement liquides contenant respectivement de l'ammoniac et du carbamate ; et des sorties (45a, 45b) respectives reliées au réacteur (2) ; dans lequel les turbines (41a, 41b) du turbocompresseur (40) définissent respectivement une turbine côté ammoniac (41a) et une turbine côté carbamate (41b) ; la turbine côté ammoniac (41a) étant entraînée par le passage du premier flux, contenant de l'ammoniac, fourni au turbocompresseur (40) à travers la conduite d'ammoniac (34) ; la turbine côté carbamate (41b) étant entraînée en rotation, par l'intermédiaire de l'arbre (42), par la turbine côté ammoniac (41a) et agissant sur le deuxième flux, contenant du carbamate, fourni à travers la conduite de carbamate (27) pour augmenter la pression de celui-ci.

2. Réacteur selon la revendication 1, dans lequel les sorties (45a, 45b) du turbocompresseur (40) sont reliées respectivement à ladite deuxième entrée (12) du réacteur (2), par l'intermédiaire de la conduite d'alimentation en ammoniac (22), et à une troisième entrée (13) du réacteur (2), par l'intermédiaire de la conduite d'alimentation en carbamate (23).

3. Réacteur selon la revendication 2, dans lequel ladite troisième entrée (13), reliée à la conduite d'alimentation en carbamate (23), est positionnée à une position différente, par exemple à une position plus haute, dans le réacteur (2) par rapport à ladite deuxième entrée (12), reliée à la conduite d'alimentation en ammoniac (22).

4. Réacteur selon l'une quelconque des revendications précédentes, dans lequel la conduite d'alimentation en ammoniac (22) se divise, en aval du turbocompresseur (40), en deux branches d'alimentation en ammoniac (47) ou plus reliées à des entrées (12) respectives du réacteur (2), de manière à alimenter le réacteur (2) en ammoniac à des positions différentes.

5. Réacteur selon l'une quelconque des revendications précédentes, dans lequel la conduite de carbamate (27) comporte une ramification (48) reliée à une conduite de carbamate auxiliaire (49) qui est reliée directement réacteur (2) et ne traverse pas le turbocompresseur (40), en particulier à une entrée supplémentaire (51) du réacteur (2).

6. Usine d'urée (1), **caractérisée en ce qu'**elle comprend le réacteur (2) et le système d'alimentation associé selon l'une quelconque des revendications précédentes.

7. Méthode d'alimentation d'un réacteur de synthèse d'urée (2) en ammoniac, en dioxyde de carbone et en réactifs non convertis récupérés en aval du réacteur (2), comprenant les étapes de l'alimentation du réacteur (2) en CO2 et en ammoniac par l'intermédiaire respectivement d'une première entrée (11) et d'une deuxième entrée (12) ; la méthode étant **caractérisée par** la fourniture d'un premier flux sensiblement liquide contenant de l'ammoniac à une première turbine (41a) qui est entraînée en rotation par ledit premier flux agissant en tant que fluide d'entraînement ; la fourniture d'un deuxième flux sensiblement liquide comprenant du carbamate à une deuxième turbine (41b), reliée solidairement à la première turbine par un arbre de rotation (42), pour augmenter la pression dudit deuxième flux ; la fourniture desdits premier et deuxième flux, après une coopération avec lesdites turbines (41a, 41b), au réacteur (2), les turbines étant d'un turbocompresseur (40) selon la revendication 1.

8. Méthode selon la revendication 7, dans laquelle les turbines (41a, 41b) définissent respectivement une turbine côté ammoniac (41a) et une turbine côté carbamate (41b) ; la turbine côté ammoniac (41a) étant entraînée par le passage dudit premier flux ; la turbine côté carbamate (41b) étant entraînée en rotation, par l'intermédiaire de l'arbre (42), par la turbine côté ammoniac (41a) et agissant sur ledit deuxième flux pour augmenter la pression de celui-ci.

9. Méthode selon la revendication 7 ou 8, dans laquelle ledit premier flux et ledit deuxième flux, après une coopération avec lesdites turbines (41a, 41b), sont fournis au réacteur (2) séparément l'un de l'autre.

10. Méthode selon la revendication 9, dans laquelle le deuxième flux, contenant du carbamate, est alimenté dans le réacteur (2) à une position différente, par exemple à une position plus haute, par rapport au premier flux.

11. Méthode selon l'une quelconque des revendications 7 à 10, comprenant une étape de division dudit premier flux, après une coopération avec ladite première turbine (41a), en deux flux ou plus qui sont alimentés au réacteur (2) à des positions différentes.

12. Méthode selon l'une quelconque des revendications 7 à 11, comprenant l'étape de la fourniture au réacteur (2) d'un autre flux sensiblement liquide contenant du carbamate séparément dudit deuxième flux et sans traverser ladite deuxième turbine (41b) .

13. Procédé de synthèse d'urée par réaction biphasique directe d'ammoniac et de dioxyde de carbone à haute température et à haute pression, comprenant une étape d'alimentation d'un réacteur de synthèse d'urée (2) en ammoniac, en dioxyde de carbone et en réactifs non convertis récupérés en aval du réacteur (2) ; **caractérisé en ce que** ladite étape d'alimentation du réacteur de synthèse d'urée (2) en ammoniac, en dioxyde de carbone et en réactifs non convertis récupérés en aval du réacteur (2) est réalisée par la méthode selon l'une quelconque des revendications 7 à 12.
